# EUROPÄISCHE PATENTSCHRIFT

(11) **EP 1 635 838 B1**
(45) Veröffentlichungstag und Bekanntmachung des Hinweises auf die Patenterteilung: **02.05.2007**
(21) Anmeldenummer: 04739573.6
(22) Anmeldetag: 03.06.2004
(51) Int. Cl.: A61K 31/519, A61P 9/12

(54) **PHOSPHODIESTERASE-5-INHIBITOREN ZUR PROPHYLAXE UND/ODER THERAPIE DER PORTALEN HYPERTONIE**
PHOSPHODIESTERASE-5-INHIBITORS FOR THE PROPHYLAXIS AND/OR TREATMENT OF PORTAL HYPERTENSION
INHIBITEURS DE LA PHOSPHODIESTERASE-5 POUR LA PROPHYLAXIE ET/OU LA THERAPIE DE L'HYPERTONIE PORTALE

(30) Priorität: 06.06.2003 DE 10325813
(43) Veröffentlichungstag der Anmeldung: 22.03.2006
(60) Teilanmeldung: 06025229.3
(73) Patentinhaber: Universitätsklinikum Freiburg, 79104 Freiburg (DE)
(72) Erfinder: KREISEL, Wolfgang, 79100 Freiburg (DE)
(74) Vertreter: Oser, Andreas
(86) Internationale Anmeldenummer: PCT/EP2004/006014
(87) Internationale Veröffentlichungsnummer: WO 2004/108062

(56) Entgegenhaltungen:
- COLLE I ET AL.: "Systemic and splanchnic hemodynamic effects of sildenafil in rats with cirrhosis and portal hypertension" HEPATOLOGY, Bd. 36, Nr. 4 part 2, Oktober 2002 (2002-10), Seite 510A, XP009040431
- COLLE I ET AL.: "Sildenafil in rats with cirrhosis and portal hypertension: Systemic and splanchnic haemodynamic effects" DIGESTIVE DISEASE WEEK ABSTRACTS AND ITINERARY PLANNER, Bd. 2003, 2003, Seite A-736, XP001203834
- NELSON GARCIA ET AL.: "Systemic and portal haemodynamic effects of sildenafil citrate" GASTROENTEROLOGY, Bd. 120, Nr. 5 Suppl. 1, April 2001 (2001-04), Seite A.378, XP009040383

## Beschreibung

Die Erfindung bezieht sich auf eine neue Medikation zur Prophylaxe und/oder Therapie bei der portalen Hypertonie (Hochdruck im Pfortaderbereich) sowie eng damit zusammenhängender Krankheiten bzw. medizinischer Komplikationen.

Die portale Hypertonie (Pfortaderhochdruck,' d.h. der Hochdruck im Bereich der Pfortader) ist unter anderem Ursache für gefürchtete Blutungen aus dem oberen Magen-Darm-Trakt. Am häufigsten handelt es sich um eine Blutung aus "Ösophagusvarizen", d.h. Krampfadern, die sich in der Speiseröhre ausbilden, weil die Strömung des Blutes durch die Leber behindert ist und sich das Blut einen "Umwegskreislauf" sucht. Eine weitere problematische Komplikation der portalen Hypertonie sind Blutungen aus Fundusvarizen. Daneben sind weitere schwerwiegende oder lebensbedrohliche Komplikationen der portalen Hypertonie bekannt (hepatische Enzephalopathie, hepatorenales Syndrom, hepatopulmonales Syndrom, Aszites, spontane bakterielle Peritonitis, Stoffwechselstörungen infolge verminderter Leberdurchblutung).

Die akute Blutungskomplikation wird mit Medikamenten (z.B. Vasopressin und -Analoga, Somatostatin und -Analoga, Angiotensin-2-Rezeptor-Antagonisten) und/oder durch endoskopische Verfahren (Ballontamponade, Sklerosierung, Varizen-Banding, Injektionstherapie mit Acryl-Harzen, TIPS (Transjugulärer portosystemischer Stent-Shunt)) therapiert. Die Primär- bzw. Sekundärprophylaxe erfolgt herkömmlich durch medikamentöse (nichtkardioselektive β-Blocker; Nitrate), endoskopische (Sklerotherapie, Varizen-Banding, TIPS) oder operative (Shunt-Operationen) Therapie. Durch die medikamentöse Prophylaxe mittels Gabe von β-Blockern bzw. Nitraten erreicht man eine Senkung des Pfortaderdrucks durch eine Senkung der Herzfrequenz, des peripheren Gefäßwiderstandes und des systemischen Blutdrucks (siehe Literatur S. Chung, J. Gastroenterol. Hepatol. 2002, 17:355-360; und G. Mc. Cormack und P.A. Mc. Cormick, Drugs 1999, 57:327-335).

Gerade am Beispiel von Nitraten zeigt sich aber das Dilemma von Wirkstoffen, die generell vasodilatorisch wirken, wie dies aus G.Garcia-Tsao: "Portal hypertension". Current Opinion in Gastroenterology, 2003, 19, S. 250-258; und R.Wiest und R.J.Groszmann: "The Paradox of Nitric Oxide in Cirrhosis and Portal Hypertension: Too Much, Not Enough", Hepatology 35, No. 2, 2002, S. 478-491, deutlich wird: Eine Senkung des peripheren Gefäßwiderstands bzw. des systemischen Blutdrucks kann den Blutstau zur Leber hin sogar erhöhen und damit die pathologische Situation sogar verschlimmern.
Das heißt, selbst eine vermutete generelle Durchblutungssteigerung bzw. Vasodilatation, die von einem Medikament hervorgerufen wird, bedeutet nicht automatisch, dass dieses Medikament eine Steigerung der Leberdurchblutung bewirkt und/oder eine Wirkung gegen die portale Hypertonie hat. Die etablierten Medikamente wie β-Blocker, Vasopressin und -derivate, Somatostatin und -derivate senken den Pfortaderdruck dadurch, dass sie den arteriellen Zustrom zur Leber drosseln. Dadurch wird allerdings die ohnehin bereits beeinträchtigte gesamte (portale) Durchblutung der Leber noch weiter verschlechtert. Daher wurde bereits seit vielen Jahren nach Substanzen gesucht, die mehr oder weniger selektiv den Pfortaderdruck senken. Insbesondere ist es erwünscht, eine Therapie anwenden zu können, durch welche die portale Durchblutung der Leber gesteigert werden kann, ohne dabei die arterielle Perfusion der Leber entscheidend zu verändern.

Es kommt zu den Problemen der medikamentösen Therapie noch hinzu, dass die portale Hypertonie eine Krankheitserscheinung mit sehr komplexer und vielfältiger Ätiologie ist und nicht mit dem pathologisch-anatomisch definierten Zustand Leberzirrhose gleichzusetzen ist. Von der Leberzirrhose als solche verschieden kann die portale Hypertonie eine Folge der Leberzirrhose, aber auch völlig anderer Ursachen sein, wie in der folgenden Aufstellung angegeben ist:
(I) Prähepatisch:
   Thrombosen im Bereich der Pfortader
   Arterio-venöse Fisteln
   Jede Erkrankung mit einer deutlich vergrößerten Milz (verstärkter Blutfluß zur Leber hin)
(II) Intrahepatisch:
   *a) Präsinusoidal:*
      Schistosomiasis (Bilharziose)
      Sarkoidose und andere granulomatöse Erkrankungen
      Primär biliäre Zirrhose (noch bevor sie in eine eigentliche Zirrhose übergegangen ist)
      Myeloproliferative Syndrome, z. B. chronische myeloische Leukämie, Osteomyelofibrose
      Lymphatische Systemerkrankungen
      Kollagenosen (z. B. systemischer Lupus erythematodes, Sklerodermie)
      Kongenitale Leberfibrose
      Leberfibrosen anderer Ursache
      M. Osler (angeborene arterio-venöse Fehlbildungen, u. a. auch in der Leber)
      "Idiopathische portale Hypertonie" (PH ohne fassbare Ursache)
   *b) Sinusoidal:*
      Leberzirrhose
      Noduläre regenerative Hyperplasie
      Toxischer Leberschaden
      Schwer verlaufende Hepatitis
   *c) Postsinusoidal:*
      Leberzirrhose
      Toxischer Leberschaden
      Lebervenenverschlußsysndrom (VOD = veno-occlusive disease)
      Noduläre regenerative Hyperplasie
      Schwer verlaufende Hepatitis
(III) Posthepatisch:
   Rechtsherzinsuffizienz
   Trikuspidalinsuffizienz
   Pericarditis constrictiva
   Budd-Chiari-Syndrom (Thrombosen der Lebervenen)
   Fehlbildungen der Lebervenen
   Kompression der Lebervenen (z. B. durch Tumoren)

Die bisherigen Prophylaxe- und Therapiekonzepte gegen die portale Hypertonie weisen keine ausreichende Erfolgsrate auf. Weder die Blutungskomplikationen noch die zahlreichen anderen Krankheiten bzw. medizinischen Komplikationen der portalen Hypertonie können bisher befriedigend therapiert oder verhütet werden.

Aufgabe der Erfindung ist es daher, die therapeutischen Möglichkeiten der Primär- und/oder Sekundärprophylaxe und/oder der Therapie der portalen Hypertonie und der damit assoziierten oder verwandten Krankheiten bzw. Komplikationen zu erweitern und zu verbessern.

Diese Aufgabe wird durch die Verwendung gemäß einem der Ansprüche 1 bis 3 gelöst. Ein weiterer Gegenstand der Erfindung ist im Anspruch 5 angegeben. Bevorzugte Weiterbildungen der erfindungsgemäßen Gegenstände sind in den Unteransprüchen definiert.

Mit der vorliegenden Erfindung wird ein völlig neuer biochemischer bzw. pharmakologischer Ansatzpunkt geschaffen, der bessere Therapiemöglichkeiten zur Prophylaxe und/oder Therapie der portalen Hypertonie und der damit assoziierten bzw. verwandten Krankheiten oder Komplikationen eröffnet. Als Grundlage für diesen neuen Ansatzpunkt und die verbesserten Therapiemöglichkeiten wurde überraschend gefunden, daß Inhibitoren der Phosphodiesterase 5 in der Lage sind, den Pfortaderdruck deutlich zu senken, den Pfortaderdurchmesser zu vergrößern und den Pfortaderfluss signifikant zu erhöhen.

Inhibitoren, die in bezug auf die Phosphodiesterase 5 (PDE-5) spezifisch sind (derzeit sind bei Menschen 11 Phosphodiesterase-Typen bekannt), sind an sich bekannt, jedoch für andere Indikationen als die der vorliegenden Erfindung bzw. in unterschiedlichem Zusammenhang untersucht.

Das bekannteste Indikationsgebiet ist die männliche Erektionsstörung (MED, Impotenz) (s. WO 94/28902 A), und der bekannteste für diesen Zweck entwickelte Wirkstoff ist die Verbindung Sildenafil (INN) (s. Übersichtsartikel über diesen und andere PDE 5-Inhibitoren zu diesem Zweck U.Gresser und C.H.Gleiter in Eur. J. Med. Res. 2002, 435-446). Chinazoline mit cGMP-Phosphordiesterase hemmender Aktivität sind z.B. in J. Med. Chem. 1993, 36:3765 ff. und in J. Med. Chem. 1994, 37:2106 ff. beschrieben. Die WO 02/060449 A beschreibt eine pharmazeutische Formulierung enthaltend einen PDE 5-Inhibitor kombiniert mit Nitraten (Pryzolo[4,3-d]Pyrimidine und Nitrate oder Thienopyrimidine und Nitrate). Die pharmazeutische Formulierung wird zur Herstellung eines Arzneimittels beschrieben, welches angeblich zur Behandlung einer Reihe unterschiedlicher Erkrankungen möglich sein könnte: Angina, Bluthochdruck, pulmonaler Hochdruck, kongestives Herzversagen (CHF), chronische obstruktive pulmonale Krankheit (COPD), Cor pulmonale, Rechtsherzinsuffizienz, Artherosklerose, Bedingungen verminderter Durchgängigkeit der Herzgefäße, periphere vaskuläre Krankheiten, Schlaganfall, Bronchitis, allergisches Asthma, chronisches Asthma, allergische Rhinitis, Glaukom, (irritables Bowel-Syndrom) Reizdarmsyndrom, Tumore, Niereninsuffizienz und Leberzirrhose. Die WO 02/062343 A2 beschreibt eine pharmazeutische Formulierung, enthaltend mindestens einen PDE 5-Inhibitor und mindestens einen Endothelin-Rezeptorantagonisten, zur Herstellung eines Medikaments, welches für die gleichen Krankheiten wie in der WO 02/060449 A und zusätzlich für Erektionsstörungen und die Behandlung von weiblichen Sexualerkrankungen vorgeschlagen wird. Die EP 1 097 711 A2 beschreibt die Verwendung bestimmter PDE 5-Inhibitoren, z.B. Sildenafil, zur Behandlung der pulmonalen Hypertonie. H.A. Ghofrani et al. (Pulmologie 56, 665-672 (2002) beschreiben Sildenafil zur Behandlung der pulmonalen Hypertonie und möglicherweise auch der beginnenden Herzinsuffizienz.

Ferner berichten N.Garcia Jr. et al. in Digestive Disease Week 2001, Abstract A-391, sowie I.Colle et al. in Digestive Disease Week 2003, Abstract No. S1553 in jeweiligen Abstracts über tierexperimentelle Untersuchungen zu systemischen bzw. splanchnischen oder portalen hämodynamischen Effekten von Sildenafil bei der Ratte. Es werden jeweils sehr artifizielle Versuchsbedingungen gewählt, indem diese Substanz i.v. bzw. intraarteriell (in die Arteria mesenterica superior) in sehr hohen Dosen (0,1, 1 und 10 mg/kg Körpergewicht bei Garcia et al. und 0,01-10 mg/kg Körpergewicht bei Colle et al.) verabreicht wurde. Es wurde beobachtet, dass in einem sehr kurzzeitigen Effekt (deutlich unter 5 Minuten) der arterielle Gefäßwiderstand abfällt; hinsichtlich des portalvenösen Drucks sind die Angaben von Garcia et al. und Colle et al. widersprüchlich. Die artifiziellen Tierexperimente deuten auf einen unphysiologisch kurzzeitigen, generellen, d.h. nicht-selektiven vasodilatorischen Effekt von Sildenafil infolge direkter intravasaler Gabe bei der Ratte hin.

Die besondere Wirksamkeit von PDE 5-Inhibitoren allein oder in Kombination mit anderen Wirkstoffen gegen die portale Hypertonie, insbesondere in Kombination mit drosselnd auf die Herzfrequenz, alternativ auf die arterielle Blutzufuhr in die Leber wirkenden Substanzen wie β-Blockern oder Vasopressin, Vasopressin-Analoga wie Terlipressin, Somatostatin oder Somatostatin-Analoga wie Octreotid zur Primär- oder Sekundärprophylaxe und/oder zur Therapie bei der portalen Hypertonie, insbesondere zur Behandlung von Blutungskomplikationen, wird in den genannten Dokumenten jedoch weder in Betracht gezogen, noch lassen hypothetische Indikationen der genannten Dokumente auf die erfindungsgemäß gefundenen, speziellen Ergebnisse einer signifikanten Senkung des Pfortaderdrucks und einer signifikanten Erhöhung des Pfortaderflusses schließen. Gegenüber den Tierexperimenten von Garcia et al. und Colle et al. wurden erfindungsgemäß qualitativ und quantitativ andere Wirkungen beim Menschen erhalten.

Die vorliegende Erfindung stellt die Verwendung von PDE 5-Inhibitoren zur Primär- oder Sekundärprophylaxe und/oder zur Therapie gegen die portale Hypertonie sowie gegen Krankheitszustände oder Risiken, die mit der portalen Hypertonie assoziiert sind, zur Verfügung. Insbesondere sind dies folgende Krankheiten: Blutungskomplikationen der portalen Hypertonie, insbesondere bei Blutungen aus Ösophagus-Varizen, Fundus-Varizen und/oder bei portal hypertensiver Gastropathie; ferner die Krankeiten hepato-renales Syndrom, hepato-pulmonales Syndrom, hepatische Enzephalopathie, spontane bakterielle Peritonitis und Aszites.
Darüber hinaus ist der Einsatz eines PDE 5-Inhibitors oder einer einen PDE 5-Inhibitor enthaltenden pharmazeutischen Zusammensetzung zur Prophylaxe und/oder Therapie gegen Leiden oder Krankheitszuständen indiziert, die durch Senkung des Pfortaderdrucks und/oder durch Erhöhung des Pfortaderflusses gemildert oder dadurch vorgebeugt werden können. Hierfür kommen weitere Krankheiten oder Krankheitszustände in Betracht, die vorgebeugt oder gelindert oder sogar geheilt werden können durch die über die Senkung des Pfortaderdrucks und/oder durch Erhöhung des Pfortaderflusses erreichte verbesserte Durchblutung der Leber. Das sind in erster Linie solche Krankheiten bzw. Krankheitszustände, die durch Störungen des Stoffwechsels oder des Blutkreislauf in Verbindung mit der Leber gekennzeichnet sind, z.B. Entgiftungsstörungen, verminderter oder gestörter Abbau von Medikamenten, falscher Substanzabbau, Entstehung eines Umwegskreislaufs um die Leber, Abwehrschwäche, Stauung des Bluts in der Milz und nachfolgende Krankheiten wie Leukocytopenie, Thrombocytopenie, Störung der Synthese von Gerinnungsfaktoren, Störungen der Hirnfunktion und dergleichen.

Die Wirkung von Inhibitoren der PDE-5 auf die Steigerung der portalen Durchblutung kann aber auch ausgenützt werden bei gesunder Leber, die durch endogene toxische Substanzen oder durch Medikamenten-, Drogen- und/oder Alkoholkonsum oder durch ähnliche exogene, insbesondere toxischen Substanzen gefährdet sein kann. Wenn eine Substanz in der Leber metabolisiert wird, was insbesondere bei den erwähnten, exogen zugeführten Substanzen und typischerweise auch bei weiteren endogenen oder exogenen Substanzarten der Fall ist, kann deren Metabolismus durch eine Steigerung der Durchblutung der Leber, die aus dem gesteigerten Zustrom aus der Pfortader ohne Druckerhöhung der Pfortader selbst resultiert, gezielt beeinflusst werden. Der metabolische Abbau der endogenen oder der exogen zugeführten Substanz in der Leber kann so verstärkt werden.

Die Substanz, deren Metabolismus beeinflusst werden soll, kann aus der Gruppe ausgewählt werden, die aus Medikamenten, Drogen oder toxischen Substanzen wie Äthylalkohol besteht. Die Beeinflussung des Metabolismus der Substanz beinhaltet eine Erhöhung des Pfortaderflusses und dadurch vorteilhafterweise eine Steigerung der Leberdurchblutung. Bei der Verwendung mit der exogen zugeführten Substanz kann diese gleichzeitig mit, nach oder insbesondere vor der Gabe des PDE 5-Inhibitors oder der einen PDE 5-Inhibitor enthaltenden pharmazeutischen Zusammensetzung eingenommen werden.

Speziell bei dem Aszites ist eine Kombination eines PDE 5-Inhibitors mit einem beliebigen Diuretikum, z.B. Furosemid (Beispiel für ein Schleifendiuretikum) oder Spironolacton (ein Aldosteron-Antagonist), besonders geeignet, um die prophylaktische oder heilende Wirkung zu verstärken.

Die Messungen von Pfortaderdruck, Pfortaderdurchmesser und Pfortaderfluss können mit bekannten Methoden ausgeführt werden, z.B. nicht-invasiv durch Doppler-sonographische Messungen (s. z.B. A.Albillos et al., J. Hepatol. 27, 496-504 (1997)), oder invasiv durch Bestimmung des hepatovenösen Druckgradienten (HVPG) (s. J.P. Tasu et al., Clin. Radiol. 57, 746-752 (2002)) oder des Lebervenenverschlussdrucks (WHVP = wedged hepatic vein pressure).

Als PDE 5-Inhibitoren sind die an sich aus den vorstehend genannten Dokumenten bekannten PDE 5-Inhibitoren und wegen der besonderen Selektivität für PDE 5 gegenüber anderen Phosphodiesterasen insbesondere die folgenden Substanzen für die erfindungsgemäße Verwendung geeignet: Sildenafil (INN), d.h. 1-{[3-(6,7-Dihydro-1-methyl-7-oxo-3-propyl-1H-pyrazolo-[4,3-d]pyrimidin-5-yl)-4-ethoxyphenyl]sulfonyl}-4-methyl(3,4-methylendioxyphenyl)-12,12a-dihydro-6H-pyrazino [1,2:1,2]-pyrido[5,4-b]indol-1,4(2H,3H)-dion, und Vardenafil (Strukturen s. U.Gresser und C.H.Gleiter oben). Hierzu gehören auch die modifizierten oder analogen Formen dieser Substanzen, die dem Fachmann bekannt sind und denen ebenfalls eine Hemmung der PDE 5 zugeschrieben wird.

Bedeutend für den überraschenden Erfolg der PDE 5-Inhibitoren als Wirkstoff speziell bei den oben beschriebenen Indikationen dürfte die im Rahmen der Erfindung beobachtete, überraschend differenzierte Selektivität der Wirkung auf die bei der portalen Hypertonie relevanten Gefäße beim Menschen sein: Während der Gefäßwiderstand der arteriellen die Leber versorgenden Gefäße unverändert bleibt oder sogar zunimmt (und entsprechend die arterielle Leberdurchblutung gleich bleibt oder abnimmt), steigt der portale Fluss signifikant an. Da speziell die physiologische arterielle und portale Leberdurchblutung komplementär sind - d.h. dass die arterielle Durchblutung sinkt, wenn die portale Durchblutung steigt, und umgekehrt - wird vermutet, dass sich aufgrund der Komplementarität die selektive Wirkung am Zielort zu der überraschenden Effektivität auswirkt.

Weil sich die Wirkungen jeweils günstig ergänzen, kann erfindungsgemäß eine Kombination vorzugsweise erfolgen durch Verwendung, in einer gemeinsamen oder in getrennten Formulierungen (Kombinationsmedikation), des PDE 5-Inhibitors in Kombination mit einem anderen Wirkstoff gegen die portale Hypertonie. Insbesondere kann der kombinierte Wirkstoff ausgewählt werden aus der Gruppe von Vasopressin und dessen Analoga wie Terlipressin und Somatostatin und dessen Analoga wie Octreotid, um zu einer synergistischen Erhöhung des arteriellen Gefäßwiderstands und somit zur Drosselung der arteriellen Blutzufuhr zu Darm und Leber beizutragen (vgl. die oben erläuterte physiologische Komplementarität der Leberdurchblutung). Alternativ sind auch die Herzfrequenz drosselnde Wirkstoffe, insbesondere β-Blocker, verwendbar. Eine Kombination des PDE 5-Inhibitors mit Nitraten/organischen Nitraten und/oder ohne Endothelin-Rezeptorantagonisten kann je nach Wunsch und Indikation entfallen, damit keine gegenläufigen Effekte oder Risiken, insbesondere im oben beschriebenen Fall der Nitrate/organischen Nitrate, auftreten.

Der PDE 5-Inhibitor als Wirkstoff kann zusammen mit einem pharmazeutisch akzeptablen Träger und/oder Streckmittel(n) und/oder Hilfsstoff(en) in zur Verabreichung menschlicher Patienten geeigneten Formulierungen enthalten sein. Der Wirkstoff oder die Zusammensetzung kann eine feste, halbfeste, gel-artige oder hydrogel-artige, eine flüssige oder eine ähnliche Konsistenz aufweisen und z.B. die Form einer Lösung, einer Suspension, eines Pulvers, einer Tablette, einer Pille, einer Kapsel, einer Verzögerungsfreisetzungsformulierung und dergleichen annehmen. Die Zusammensetzungen enthalten geeigneterweise eine wirksame therapeutische Menge des PDE5-Inhibitors zusammen mit einer geeigneten Menge eines Trägers oder Vehikels, um die Form zur passenden Verabreichung für den Patienten bereitzustellen.

Solche Formulierungen können z.B. allein oder in Kombination enthalten: geeignete pharmazeutische Zusatzstoffe wie Stärke, Cellulose und Cellulose-Derivate, Glucose, Lactose, Sucrose, Gelatine, Silicagel, Magnesiumcarbonat, Magnesiumstearat, Natriumstearat, Glycerolmonostearat, Talk, Natriumchlorid, getrocknete entrahmte Milch, Glycerin, Propylen, Glykol, Wasser, Ethanol oder ein anderer Alkohol und dergleichen; sterile Flüssigkeiten wie Wasser, Salzlösungen und wässrige Dextrose- und Glycerollösungen, und/oder Öle, einschließlich Petroleumöle, tierischen, pflanzlichen oder synthetischen Ursprungs wie Erdnussöl, Sojabohnenöl, Mineralöl, Sesamöl und dergleichen. Die Applikationsform kann eine typische Applikationsform sein, zum Beispiel oral, parenteral, insbesondere zur Injektion, ein Transdermalsystem, nasal oder zur Inhalierung. Für akute Blutungen kommen insbesondere intravenöse (i.v.) Injektionen eines PDE 5-Inhibitors und vorzugsweise in Kombination mit einem die Herzfrequenz, alternativ die arterielle Leberblutzufuhr drosselndem Wirkstoff in Betracht, wie mit β-Blockern, alternativ mit Vasopressin oder Somatostatin bzw. deren Derivate.

Zur Applikation ist der PDE5-Inhibitor und ggf. der genannte Kombinationswirkstoff geeigneterweise in einer der oben genannten Zusammensetzungen, Formulierungen oder Einrichtungen, und zwar in einer für den Menschen zur Prophylaxe oder zur Therapie der oben genannten Krankheiten oder Krankheitszuständen wirksamen Menge enthalten. Zum Beispiel beträgt eine geeignete Menge, bezogen auf die Gesamtmenge, jeweils mindestens 0,0001 Gew.-%, normalerweise 0,01 bis 20 Gew.-%, vorzugsweise 0,01 bis 10 Gew.-%. Als Dosis ist eine Menge des Wirkstoffs von mindestens 0,0001 mg/kg, z.B. in einem Bereich von 0,0001 bis 1000 mg/kg, vorzugsweise 0,01 bis 100 mg/kg und insbesondere 0,1 bis 20 mg/kg Körpergewicht des behandelten Patienten geeignet, wobei die einzelne Dosis jeweils ein bis mehrere Male täglich oder wöchentlich, bei Depot- oder Slow-Release-Formulierungen ggf. auch weniger, wie beschrieben lokal bzw. systemisch appliziert wird.

Es hat sich gezeigt, dass die Wirkungen des PDE5-Inhibitors je nach Applikationsart und Dosis unterschiedlich sein können. Überraschenderweise wurde eine besonders selektive Wirkung des PDE5-Inhibitors auf die portale Hypertonie beobachtet, wenn der PDE 5-Inhibitor bzw. die einen PDE 5-Inhibitor enthaltende pharmazeutische Zusammensetzung oral verabreicht wurde, und insbesondere dann, wenn die Dosis niedrig bis moderat ist, z.B. bei einer jeweils verabreichten Dosis des PDE 5-Inhibitors von 0,01 bis 10 und insbesondere 0,1 bis 1,5 mg PDE 5-Inhibitor pro kg Körpergewicht. Zum Vergleich: Eine nach solcher oraler Verabreichung erreichte maximale Serumkonzentration des PDE 5-Inhibitors liegt deutlich unter der Serumkonzentration dieses PDE 5-Inhibitors, der infolge Injektion oder Infusion (i.v., i.a. oder i.m.) des PDE5-Inhibitors mit einer Konzentration von 0,01 mg/kg Körpergewicht erreicht wird. Es kann vermutet werden, dass die besonders effektive Wirkung des PDE5-Inhibitors bei oraler Gabe der beschriebenen Dosis auf einem "First-Pass"-Effekt durch die Leber beruht.
Wenn dennoch eine Injektion als Applikationsform in Betracht gezogen wird, so sollte die Dosiskonzentration vorzugsweise entsprechend niedrig gewählt werden, z.B. 0,01 mg/kg Körpergewicht.
Dementsprechend liegt im Allgemeinen die Dosis pro Gabe vorteilhafterweise in einem Bereich, in dem eine selektive Wirkung auf die Zielgefäße (z.B. bestimmbar durch Erhöhung des portalen Blutflusses und gleichzeitig gleichbleibendem oder ansteigendem Widerstand (definiert als Resistivity Index Ri) sicher gewährleistet ist, z.B. weniger als 1,5 (weiter bevorzugt weniger als 1,0) mg/kg Körpergewicht bei oraler Gabe, weniger als 0,01 (weiter bevorzugt weniger als 0,005) mg/kg Körpergewicht bei Injektion oder Inhalierung, und weniger als 0,005 (weiter bevorzugt weniger als 0,001) mg/kg Körpergewicht pro Minute bei Infusionen.

Bezüglich der kombinierten Wirkstoffe oder der metabolisch abzubauenden, exogen zugeführten Substanz der erfindungsgemäßen Kombinations-Medikation oder -Präparation kann der Fachmann je nach Anwendungsfall und Bedarf die Applikationsformen, -Zusammensetzungen und -mengen wählen, die für die jeweiligen kombinierten Wirkstoffe oder die exogen zugeführten Substanzen üblich sind. Unabhängig von der PDE 5-Applikationsform kommen daher geeignete pharmazeutisch akzeptable Träger, Streckmittel, Hilfsstoffe etc. und passende Formulierungen für die ggf. kombinierten Wirkstoffe der Kombinationsmedikation bzw. -präparation in Betracht.

Die Herstellung eines Medikaments bzw. einer Kombinationsmedikation sowie die medizinische Anwendung zur Prophylaxe und/oder Therapie ergeben sich für den Fachmann ohne weiteres aus der obigen Beschreibung.

Die besondere Wirksamkeit von PDE 5-Inhibitoren für die oben genannten, speziellen Indikationsgebiete ergibt sich wie folgt:

### Portale Hypertonie,

### Blutungskomplikationen, etc.:

In einer Pilotstudie wurde teilweise eine deutliche Vergrößerung des Durchmessers der Pfortader und der Milzvene festgestellt. Die maximale Flußgeschwindigkeit des Bluts in der Pfortader und in der Milzvene nahm nur gering ab. Der Widerstand der arteriellen Durchblutung der Leber zeigte praktisch keine Veränderung, oder er nahm sogar zu. Demgegenüber nahm der Pfortaderfluß durch die Leber deutlich zu (z.B. teilweise um über 30%).

Durch die Druckminderung und die Förderung des Flusses im Pfortaderkreislauf kann somit der Bildung von Kollateralen zwischen Pfortadersystem und venösem Gefäßsystem und somit der Bildung von Ösophagus-Varizen und/oder Fundus-Varizen vorgebeugt werden, bzw. durch eine Senkung des Pfortaderdrucks durch Gabe von Inhibitoren der PDE 5 kann das Auftreten einer Blutung oder eines Blutungsrezidivs verhindert werden. Durch das rasche physiologische Ansprechen und dem Herbeiführen des Effekts "Senkung des Pfortaderdrucks" kurzzeitig oder unmittelbar nach Einnahme der PDE 5-Inhibitoren ist auch eine Therapie der akuten Blutungskomplikation möglich.

### Hepato-renales Syndrom:

Diese Krankheit stellt eine Verschlechterung der Nierenfunktion mit einer Regulationsstörung dar, obwohl die Niere selbst intakt ist. Die herkömmliche Therapie, nämlich Volumengabe, Gabe von Albumin oder von Terlipressin, ist nicht zufriedenstellend.

Erfindungsgemäß wird durch Verwendung von PDE 5-Inhibitoren der Blutfluß durch die Leber gesteigert, so daß zu erwarten ist, daß Blut aus der Pfortader dem systemischen Kreislauf zugeführt und somit eine Verbesserung des hepato-renalen Syndroms zu erwarten ist.

### Hepato-pulmonales Syndrom:

Diese Erkrankung ist mit einer portalen Hypertonie und einer arteriellen Sauerstoffuntersättigung assoziiert. Symptome sind Atemnot und nachlassende Leistungsfähigkeit. Es treten hier Kurzschlußverbindungen zwischen den Ästen der Lungenarterien und den Lungenvenen auf. Daher wird das sauerstoffarme Blut teilweise nicht wieder in der Lunge mit Sauerstoff angereichert.

Durch die erfindungsgemäße Senkung des Pfortaderdrucks und damit der positiven Wirkung auf die portale Hypertonie ist hier auch eine Verbesserung des hepato-pulmonalen Syndroms zu erwarten.

### Hepatische Enzephalopatie:

Diese Erkrankung ist ein ebenfalls mit der portalen Hypertonie assoziierter Krankheitszustand mit nicht entzündlichen Hirnschäden oder -erkrankungen. Durch die verminderte portale Durchblutung wird das Blut aus dem Gastrointestinaltrakt in der Leber weniger entgiftet, insbesondere wenn gleichzeitig ein Kollateralkreislauf vorliegt. Auch hier wird eine deutlich positive Wirkung erwartet, da durch deutliche Verbesserung der Leberdurchblutung weniger Blut an der Leber vorbei fließt und das Blut somit besser entgiftet werden kann.

Weitere Krankheiten oder Krankheitszustände, deren Situation durch die Senkung des Pfortaderdrucks und/oder durch Erhöhung des Pfortaderflusses verbessert wird:

Entsprechend gibt es eine Reihe von Krankheiten bzw. Krankheitszuständen, die durch eine verbesserte Durchblutung der Leber vorgebeugt oder gelindert oder gar geheilt werden können. Denn die Ursache solcher Krankheiten bildet gerade die mit der portalen Hypertonie einhergehende Durchblutungsstörung oder Stoffwechselstörung der Leber bzw. der gebildete Umwegskreislauf um die Leber. Daher gehören zu diesen weiteren Krankheiten bzw. Krankheitszustände z.B. Entgiftungsstörungen, verminderter oder gestörter Abbau von Medikamenten, falscher Substanzabbau, Entstehung eines Umwegskreislaufs um die Leber, Abwehrschwäche, Störung der Synthese von Gerinnungsfaktoren, Störungen der Hirnfunktion, Stauung des Bluts in der Milz und nachfolgende Krankheiten wie Leukocytopenie, Thrombocytopenie, etc..
Durch die Senkung des Pfortaderdrucks und/oder durch Erhöhung des Pfortaderflusses wird sich daher der Zustand solcher oder ähnlicher Krankheiten erwartungsgemäß bessern.

### Weitere Kombinations-Konzepte

Wenn infolge der Erhöhung des Pfortaderflusses vorteilhafterweise eine Steigerung der Leberdurchblutung einhergeht, ist zu erwarten, dass auch bei der normalen, gesunden Leber der Metabolismus einer in der Leber metabolisierten Substanz beeinflusst wird. Zu den auf diese Weise im Metabolismus beeinflussten Substanzen gehören endogene toxische Substanzen und insbesondere exogen zugeführte Substanzen wie Medikamente, Drogen oder toxische Substanzen wie Äthylalkohol. Die negativen Wirkungen dieser Substanzen auf den Organismus können dadurch abgemildert oder beseitigt werden. Ferner lässt sich auf diese Weise bei einer Kombinationsmedikation des PDE 5-Inhibitors mit einer anderen therapeutischen Substanz die Pharmakokinetik dieser kombinierten therapeutischen Substanz gezielt beeinflussen.
Solche weiteren Kombinations-Medikationen oder -Präparationen erweitern daher vorteilhaft die Therapiekonzepte völlig anderer Krankheiten, die jeweils mit der kombinierten therapeutischen Substanz zusammenhängen, und zwar unabhängig vom Vorliegen oder der potentiellen Gefahr einer portalen Hypertonie.

### Aszites und spontane bakterielle Peritonitis:

Durch Absenkung des Drucks im Pfortadersystem wird sich der Zustand des Aszites erwartungsgemäß bessern.

Die Erfindung wird nachstehend durch repräsentative Beispiele durch Verwendung eines beispielhaften PDE 5-Inhibitors näher erläutert.

### Beispiele

### 1. Beispielsserie

Bei zehn freiwilligen Probanden wurden am Morgen vor dem Frühstück die Basiswerte der Leberdurchblutung durch übliche Ultraschall- bzw. Doppler-Ultraschall-Methoden gemessen. Danach wurden pro Proband 100 mg Sildenafil oral verabreicht. Im Durchschnitt 90 Min. nach Einnahme dieses Wirkstoffs wurden die Messungen wiederholt.

Die Meßparameter und die Ergebnisse sind in der nachfolgenden Tabelle zusammengefasst.

Die Ergebnisse zeigen eine geringe Absenkung des arteriellen Blutdrucks und eine deutliche Zunahme des Durchmessers von Pfortader und Milzvene. Die maximalen Flussgeschwindigkeiten des Blutes in der Pfortader bzw. der Milzvene nahmen praktisch nicht ab, auch die Werte für den Widerstand der arteriellen Gefäße Arteria hepatica communis und Truncus coeliacus änderten sich kaum. Dagegen nahm der Fluss des Blutes in der Pfortader um 32,5 % zu. Hieraus kann bei Gabe einer wirksamen Menge eines repräsentativen Phosphodiesterase 5-Inhibitors auf eine Senkung des Drucks in der Pfortader geschlossen werden.

**TABELLE**

| Messparameter (Einheit) | MW | SD | Änderung durch Sildenafil (absolut) | Änderung durch Sildenafil (in %) |
|---|---|---|---|---|
| Systolischer Blut-druck (mm Hg) | 122 | 10,5 | -12,21 | -10,0 |
| Diastolischer Blutdruck (mm Hg) | 80,5 | 5,5 | -7,64 | -9,5 |
| Durchmesser der Pfortader (mm) | 11,7 | 0,9 | 1,5 | +12,8 |
| Vmax der Pfortader (cm/sec) | 30,5 | 7,9 | -1,63 | -5,3 |
| Durchmesser der V. lienalis (mm) | 7,7 | 0,7 | 1,0 | +13 |
| Vmax der V. lienalis (cm/sec) | 20,14 | 4,4 | -0,47 | -2,3 |
| Resistivity Index der A. hepatica communis | 0,67 | 0,07 | +0,03 | +4,48 |
| Resistivity Index des Truncus coeliacus | 0,75 | 0,05 | -0,115 | -1,5 |
| Pfortaderfluß (l/mm) | 0,83 | 0,22 | + 0,27 | +32,5 |

| | | | | |
|---|---|---|---|---|
| MV: Mittelwert SD: Standardabweichung Vmax: maximale Flußgeschwindigkeit n = 10 (bei Pfortaderfluß n = 5) | | | | |

### 2. Beispielsserie

In einer weiteren Studie wurden 16 gesunde Probanden und 15 Patienten mit portaler Hypertonie (Ursache: Leberzirrhose im Stadium Child A) folgender Behandlung unterzogen:

Bei den Probanden und Patienten wurden nüchtern (10 Stunden Nahrungskarenz) zunächst Pulsfrequenz und Blutdruck im Liegen, anschließend die Parameter der Leber-Hämodynamik durch Duplex-Sonographie gemessen.

### Duplexsonographie:

Der Pfortaderdruck wird durch Anwendung des Geräts Siemens Elegra mit einem 3,5 MHZ Sektorscanner aus den gewonnenen Werte abgeleitet (K. Haag et al., Am. J. Roentgenol 1999; 172: 631 - 635). Jede Messung wurde insgesamt drei Mal durchgeführt. Der Mittelwert aus allen drei Messungen wurde verwendet. Die Messungen wurden in Atemmittellage durchgeführt.
Bei Messungen der Flüsse und des Flussvolumens lag der Anschallwinkel unter 60°. Die Schnittebene wurde grundsätzlich so gewählt, dass das Gefäß in seinem Längsverlauf zur Darstellung kommt.

### Folgende Messpunkte und Messungen wurden vorgegeben:

### Vena portae:

Darstellung im Verlauf des Ligamentum hepatoduodenale, in der Schnittebene vom Confluens venosum bis zur Aufteilung in den rechten und linken Pfortaderhauptstamm.
Kalibermessungen der Vena portae.
Flussmessungen in der Vena portae sowie in ihren intrahepatischen Hauptstämmen.
Die Bestimmung des Flussvolumens in der Pfortader, Vena lienalis und Vena mesenterica erfolgte duplexsonographisch, ermittelt durch die Optionen des Gerätes und rechnerisch anhand der Formel: Fluss = π * d²/4 * Vₘₐₓ/2 * 60, wobei d in cm und Vₘₐₓ in cm/s angegeben wird.

### Vena lienalis:

Darstellung im Querschnitt im mittleren Oberbauch, Messung von Kaliber und Fluss.

### Vena mesenterica superior:

Darstellung entlang ihrem Verlauf vom Confluens venosum, Messung von Kaliber und Fluss.

### Truncus coeliacus, Arteria lienalis, Arteria hepatica communis:

Darstellung im Querschnitt im mittleren Oberbauch, Messung wenige Zentimeter unterhalb der Gabelung, Bestimmung des Pourcelot-Index (Ri = (V_{max[sys]} - V_{max[diast]}) / V_{max[sys]}).
Bei der Arteria hepatica communis wurde insbesondere die Flussrichtung dokumentiert zum Aussschluß einer relevanten Stenose des Truncus (hier würde eine Flussumkehr vorliegen).

### Einnahme des Medikaments:

Die Studienteilnehmer erhielten jeweils 10 mg Vardenafil oral. Nach einer Stunde wurde Blutdruck und Pulsfrequenz im Liegen gemessen, 10 ml Serum zur Spiegelkontrolle von Vardenafil entnommen, und eine erneute Messung der Leberhämodynamik durch Duplex-Sonographie (wie bei der oben angegebenen Anfangsmessung) vorgenommen.
Eine Abschlussuntersuchung zur Messung der Leberhämodynamik nach Abklingen der Wirkung von Vardenafil erfolgte nach einer Latenz von mindestens 24 Stunden nach der Medikamenteneinnahme.

### Ergebnisse:

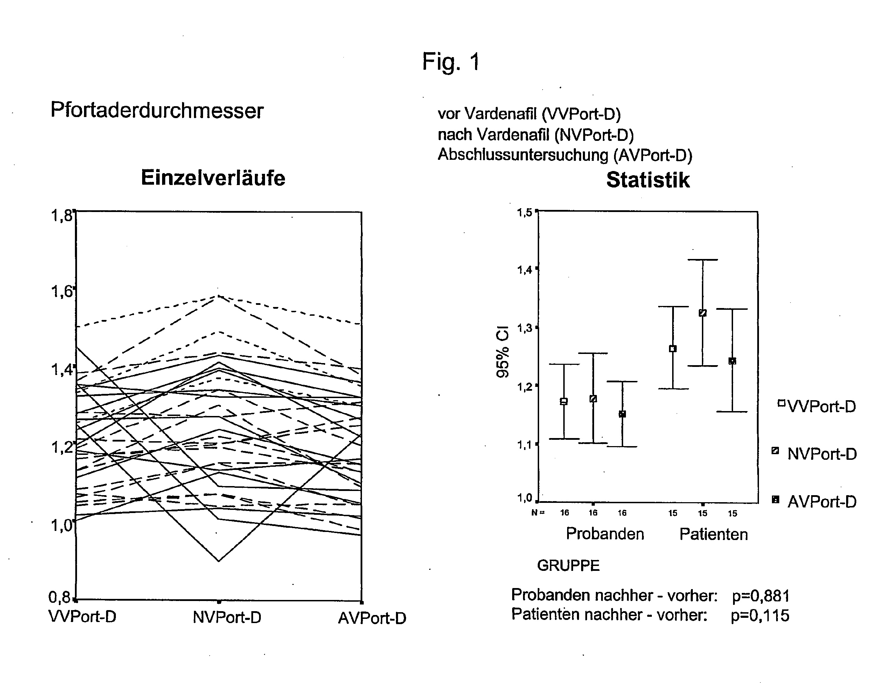

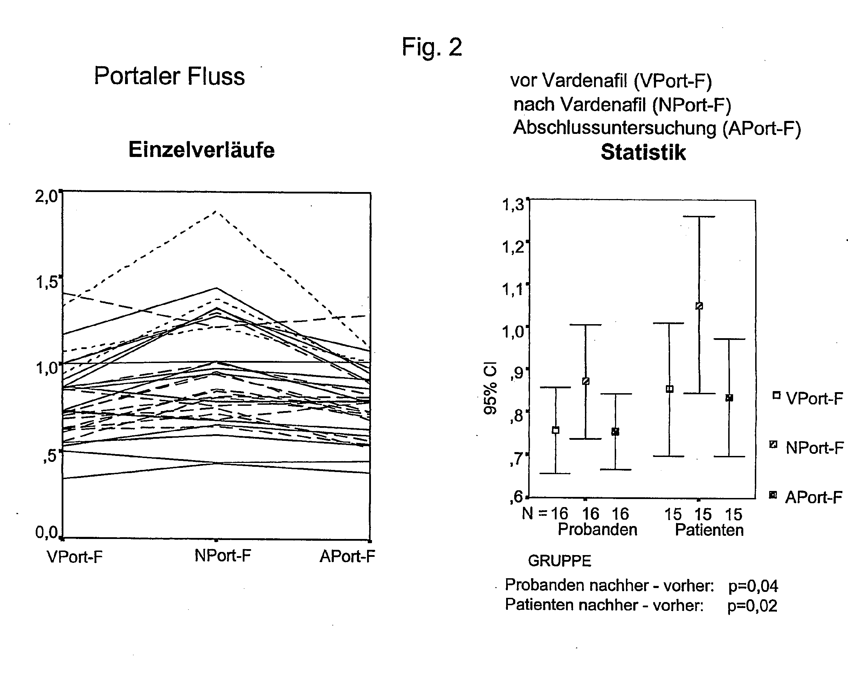

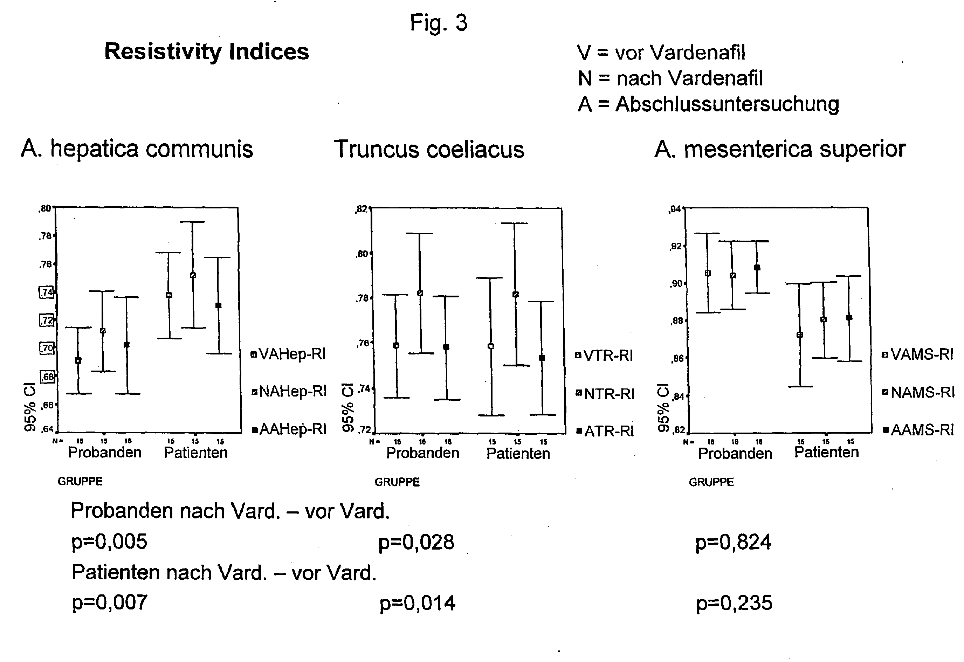

Die Ergebnisse der hämodynamischen Messungen vor (V), 1h nach oraler Gabe (N) und bei der Abschlussuntersuchung (A) sind in den Figuren 1 bis 3 gezeigt. Der Pfortaderdurchmesser nimmt bei den Patienten gering zu (Fig. 1). Er bleibt bei den normalen Probanden gleich. Durch den PDE 5-Inhibitor nimmt überraschend der portale Fluss bei den Probanden und noch deutlicher bei den Patienten signifikant zu (Fig. 2), während die arterielle Leberdurchblutung nicht verstärkt wird (Fig. 3); man sieht sogar einen Anstieg der Ri-Werte für die A.hepatica und den Truncus coeliacus. Dagegen bleiben die Ri-Werte der A.mesenterica superior unbeeinflusst.

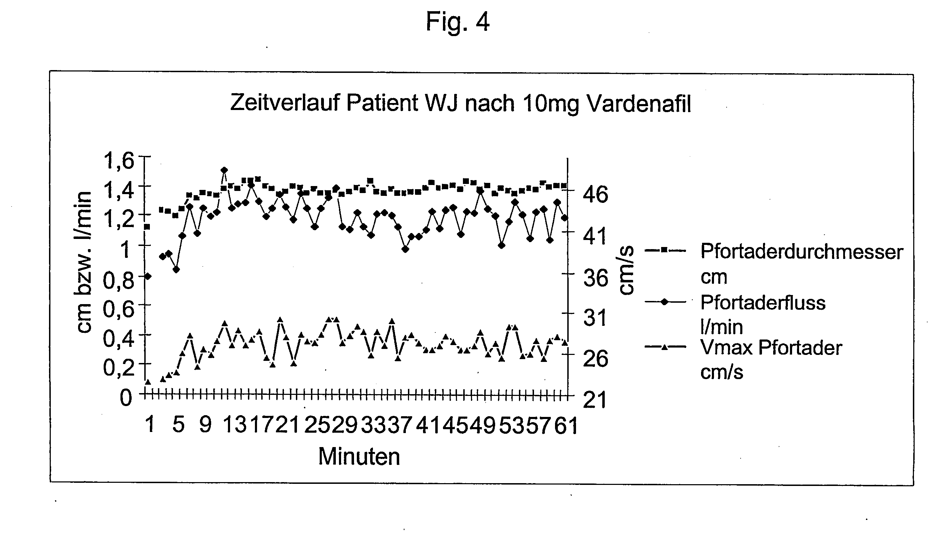

Fig. 4 zeigt ein Beispiel einer kontinuierlichen Messung bei einem individuellen Patienten (Ursache portaler Hypertonie: Leberzirrhose) nach oraler Gabe von 10 mg Vardenafil. Ähnlich wie bei praktisch allen anderen Patienten ist der Anstieg der Flussgeschwindigkeit in der Pfortader (cm/sec) und vor allem des Pfortaderflusses (1/min) schon nach etwa 15 min deutlich feststellbar. Der Effekt scheint nach 20-30 min am ausgeprägtesten zu sein, hält jedoch auch darüber hinaus für mindestens eine Stunde an.

### Beispiel 3

Gemäß der in der 2. Beispielsserie beschriebenen Studie wurden die Effekte der oralen Gabe von Vardenafil bei zwei Patienten durchgeführt, die, ursächlich auf Leberzirrhose zurückführend, einen pathologischen Kolateral- bzw. Umwegskreislauf aufwiesen. Dabei wurden vor und nach einmaliger oraler Gabe von 10 mg Vardenafil der Fluss im portosystemischen Umwegskreislauf Doppler-sonographisch gemessen: einmal bei einer Patientin mit V. coronaria ventriculi und Ösophagusvarizen, ein anderes Mal mit einer Patientin mit wiedereröffneter V. umbilicalis.
Als ein Ergebnis wurde gefunden, dass der gesamte Fluss in der Pfortader anstieg, während der Fluss in der V. coronaria ventriculi bzw. in der Nabelvene um etwa 30% absank.

An diesem Ergebnis zeigt sich der überaus günstige Effekt, dass durch den Anstieg des portalen Flusses bei unverändertem arteriellem Zufluss der portale Druck abfallen konnte und in der Folge der Umwegskreislauf zurückgedrängt werden konnte. Durch den Abfall des Flusses durch den Umwegskreislauf sinkt das Risiko von Varizenblutungen sowie von weiteren, mit dem Vorhandensein des Umwegskreislaufs assoziierten Komplikationen, Erkrankungen bzw. Krankheitszuständen.

### Beispiel 4

Bei einem Patienten mit Leberzirrhose wegen einer chronischen Hepatitis C wurde der Lebervenenverschlussdruck, der mit dem Pfortaderdruck gleichgesetzt werden kann, invasiv gemessen. Hierzu wurde über die Vena jugularis interna ein Ballonkatheter in eine kleine Lebervene unter Röntgenkontrolle eingeführt. Man kann dann den freien Lebervenendruck messen, wenn die Katheterspitze frei im Lumen der kleinen Vene liegt. Wenn man den Ballon aufbläst (der Durchmesser dieser kleinen Lebervene beträgt wenige mm), baut sich ein Staudruck an der Katheterspitze auf, der dem Pfortaderdruck entspricht. Bei dem untersuchten Patienten lag der Pfortaderdruck vor oraler Gabe eines Medikaments bei 31 mm Hg. Er zeigte bereits 10 Minuten nach der Gabe von 10 mg Vardenafil eine Tendenz, abzusinken und lag 25 Minuten nach der Einnahme der Substanz bei 26 mm Hg.
Damit wurde durch direkte Druckmessung bestätigt, dass die Schlussfolgerungen aus den indirekten Messungen der Leberhämodynamik richtig sind: Durch Inhibitoren der PDE 5 wird bei Patienten mit portaler Hypertonie der Pfortaderdruck gesenkt.

## Patentansprüche

1. Verwendung eines Inhibitors der Phosphodiesterase Typ 5 (PDE 5) oder einer PDE 5-Inhibitor enthaltenden pharmazeutischen Zusammensetzung zur Herstellung eines Medikaments zur Prophylaxe und/oder Therapie der portalen Hypertonie.

2. Verwendung eines Inhibitors der Phosphodiesterase Typ 5 (PDE 5) oder einer PDE 5-Inhibitor enthaltenden pharmazeutischen Zusammensetzung zur Herstellung eines Medikaments zur Prophylaxe und/oder Therapie gegen eine oder mehrere der folgenden Erkrankungen bzw. Komplikationen: Blutungskomplikationen der portalen Hypertonie, hepato-renales Syndrom, hepato-pulmonales Syndrom, hepatische Enzephalopatie, spontane bakterielle Peritonitis und Aszites.

3. Verwendung eines PDE 5-Inhibitors oder einer einen PDE 5-Inhibitor enthaltenden pharmazeutischen Zusammensetzung zur Herstellung eines Medikaments zur Prophylaxe und/oder Therapie gegen Leiden oder Krankheitszustände, die durch Senkung des Pfortaderdrucks und/oder durch Erhöhung des Pfortaderflusses gemildert oder **dadurch** vorgebeugt werden können.

4. Verwendung gemäß einem der Ansprüche 1 bis 3 gegen Blutungen aus Ösophagus-Varizen und/oder Fundus-Varizen.

5. Verwendung eines PDE 5-Inhibitors oder einer einen PDE 5-Inhibitor enthaltenden pharmazeutischen Zusammensetzung zur Herstellung eines Medikaments zur Beeinflussung des Metabolismus einer Substanz, wobei die Beeinflussung eine Erhöhung des Pfortaderflusses und **dadurch** eine Steigerung der Leberdurchblutung beinhaltet.

6. Verwendung gemäß Anspruch 5, wobei die Beeinflussung einen verstärkten metabolischen Abbau der exogen zugeführten Substanz in der Leber beinhaltet.

7. Verwendung gemäß Anspruch 5 oder 6, wobei die Substanz eine exogen zugeführte Substanz ist, die gleichzeitig mit, nach oder insbesondere vor der Verwendung des PDE 5-Inhibitors oder der einen PDE 5-Inhibitor enthaltenden pharmazeutischen Zusammensetzung einzunehmen ist, und/oder wobei die exogene Substanz aus der Gruppe ausgewählt wird, die aus Medikamenten, Drogen oder toxischen Substanzen wie Äthylalkohol besteht.

8. Verwendung gemäß einem der Ansprüche 1 bis 7, wobei der PDE 5-Inhibitor aus einer Gruppe ausgewählt wird, die aus Sildenafil, Tadalafil und Vardenafil besteht.

9. Verwendung gemäß einem der vorangehenden Ansprüche zur Prophylaxe und/oder Therapie beim Menschen.

10. Verwendung gemäß einem der vorangehenden Ansprüche, wobei der PDE 5-Inhibitor oder die einen PDE 5-Inhibitor enthaltende pharmazeutische Zusammensetzung oral verabreicht wird.

11. Verwendung gemäß Anspruch 10, wobei die Einzeldosis des PDE 5-Inhibitors bei der oralen Verabreichung 0,01 bis 10, insbesondere 0,1 bis 1,5 mg PDE 5-Inhibitor pro kg Körpergewicht beträgt.

12. Verwendung gemäß einem der Ansprüche 1 bis 11 in Kombination mit einer Substanz, die ausgewählt wird aus der Gruppe von β-Blockern, Vasopressin und dessen Analoga und Somatostatin und dessen Analoga.

## Claims

1. A use of an inhibitor of phosphodiesterase type 5 (PDE 5), or of a PDE 5 inhibitor-containing pharmaceutical composition, for the preparation of a medicament for prophylaxis and/or therapy of portal hypertension.

2. A use of an inhibitor of phosphodiesterase type 5 (PDE 5), or of a PDE 5 inhibitor-containing pharmaceutical composition, for the preparation of a medicament for prophylaxis and/or therapy against one or more of the following diseases or complications: bleeding complications of the portal hypertension, hepato-renal syndrome, hepato-pulmonal syndrome, hepatic encephalopathy, spontaneous bacterial peritonitis and ascites.

3. A use of a PDE 5 inhibitor, or of a PDE 5 inhibitor-containing pharmaceutical composition, for the preparation of a medicament for prophylaxis and/or therapy against illnesses or disease conditions, which can be controlled by a decrease of the portal vein pressure and/or by an increase of the portal vein flow, or which can be prevented thereby.

4. The use according to any one of claims 1 to 3 against bleedings from oesophagus varices and/or fundus varices.

5. A use of a PDE 5 inhibitor, or of a PDE 5 inhibitor-containing pharmaceutical composition, for the preparation of a medicament for influencing the metabolism of a substance, wherein the influencing involves an increase of the portal vein flow and thus an increase of a blood flow through the liver.

6. The use according to claim 5, wherein the influencing involves an enhanced metabolic decomposition of the exogenously added substance in the liver.

7. The use according to claim 5 or 6, wherein the substance is an exogenously added substance, which is incorporated concurrently with, after, or particularly prior to the use of the PDE 5 inhibitor or the PDE 5 inhibitor-containing pharmaceutical composition, and/or wherein the exogenous substance is selected from the group consisting of medicaments, drugs or toxic substances such as ethyl alcohol.

8. The use according to any one of claims 1 to 7, wherein the PDE 5 inhibitor is selected from the group consisting of Sildenafil, Tadalafil and Vardenafil.

9. The use according to any one of the preceding claims for the prophylaxis and/or therapy in humans.

10. The use according to any one of the preceding claims, wherein the PDE 5 inhibitor or the PDE 5 inhibitor-containing pharmaceutical composition is administered orally.

11. The use according to claim 10, wherein the single dosis of the PDE 5 inhibitor in the oral administration is 0.01 to 10, particularly 0.1 to 1.5 mg PDE 5 inhibitor per kg body weight.

12. The use according to any one of claims 1 to 11 in combination with a substance, which is selected from the group of β-blockers, Vasopressin and analogs thereof as well as Somatostatin and analogs thereof.

## Revendications

1. Utilisation d'un inhibiteur de la phosphodiestérase de type 5 (PDE 5) ou une composition pharmaceutique contenant un inhibiteur PDE-5, pour la fabrication d'un médicament pour la prophylaxie et/ou la thérapie de l'hypertension portale.

2. Utilisation d'un inhibiteur de la phosphodiestérase de type 5 (PDE 5) ou d'une composition pharmaceutique contenant un inhibiteur PDE 5 pour la fabrication d'un médicament pour la prophylaxie et/ou la thérapie d'une ou de plusieurs des maladies, respectivement complications suivantes : hémorragie de l'hypertension portale, syndrome hépatorénal, syndrome hépato-pulmonaire, encéphalopathie hépatique, péritonite bactérienne spontanée et l'ascite.

3. Utilisation d'un inhibiteur PDE 5 ou d'une composition pharmaceutique contenant un inhibiteur PDE 5 pour la fabrication d'un médicament pour la prophylaxie et/ou la thérapie contre des souffrances ou des maladies qui peuvent être soulagées ou prévenues par la diminution de la pression de la veine porte et/ou l'augmentation du flux de la veine porte.

4. Utilisation selon une des revendications 1 à 3 contre des hémorragies de varices de l'oesophage et/ou des varices de fond.

5. Utilisation d'un inhibiteur PDE 5 ou d'une composition pharmaceutique contenant un inhibiteur PDE-5, pour la fabrication d'un médicament destiné à influencer le métabolisme d'une substance ; cette influence comprenant une augmentation du flux de la veine porte et donc une hausse de la circulation sanguine du foie.

6. Utilisation selon la revendication 5, où cette influence comprend une décomposition métabolique renforcée de la substance amenée de façon exogène dans le foie.

7. Utilisation selon la revendication 5 ou 6, où la substance d'une substance amenée de façon exogène doit être administrée en même temps, après ou particulièrement avant l'utilisation de l'inhibiteur PDE 5 ou d'une composition pharmaceutique contenant un inhibiteur PDE 5, et/ou la substance exogène est choisie parmi le groupe constitué de médicaments, drogues ou de substances toxiques comme l'alcool éthylique.

8. Utilisation selon une des revendications 1 à 7, où l'inhibiteur PDE 5 est choisi parmi un groupe constitué de sildénafil, tadalafil et vardenafil.

9. Utilisation selon une des revendications précédentes pour la prophylaxie et/ou thérapie chez les êtres humains.

10. Utilisation selon une des revendications précédentes, où l'inhibiteur PDE 5 ou la composition pharmaceutique contenant un inhibiteur PDE 5 est administré oralement.

11. Utilisation selon la revendication 10, où la dose unique de l'inhibiteur PDE 5 s'élève de 0,01 à 10, notamment 0,1 à 1,5 mg d'inhibiteur PDE 5 par kg de poids corporel.

12. Utilisation selon une des revendications 1 à 11 en combinaison avec une substance qui est choisie parmi le groupe de béta - bloqueurs, vasopressine et ses analogues et somatostatine et ses analogues.
